# EUROPÄISCHE PATENTSCHRIFT

(11) **EP 4 126 377 B1**
(45) Veröffentlichungstag und Bekanntmachung des Hinweises auf die Patenterteilung: **20.12.2023**
(21) Anmeldenummer: 21718035.5
(22) Anmeldetag: 29.03.2021
(51) Int. Cl.: B04B 5/04

(54) **EINSATZ FÜR EINEN ROTOR EINER ZENTRIFUGE**
USE FOR A ROTOR OF A CENTRIFUGE
UTILISATION D'UN ROTOR D'UNE CENTRIFUGEUSE

(30) Priorität: 02.04.2020 DE 102020109189
(43) Veröffentlichungstag der Anmeldung: 08.02.2023
(73) Patentinhaber: Andreas Hettich GmbH & Co KG, 78532 Tuttlingen (DE)
(72) Erfinder: SCHMAL, Thomas, 78576 Emmingen (DE); GEISELMANN, Marcellus, 78532 Tuttlingen (DE); EBERLE, Klaus-Guenter, 78532 Tuttlingen (DE)
(74) Vertreter: Puschmann Borchert Kaiser Klettner Patentanwälte Partnerschaft mbB
(86) Internationale Anmeldenummer: PCT/EP2021/058069
(87) Internationale Veröffentlichungsnummer: WO 2021/198128

(56) Entgegenhaltungen:
- WO-A2-02/053292
- DE-U1-202019 104 978
- US-A- 3 559 880
- US-A- 4 720 284

## Beschreibung

Die Erfindung betrifft einen Einsatz für einen um eine Rotorachse drehbaren Rotor einer Zentrifuge gemäß der im Oberbegriff des Patentanspruches 1 angegebenen Art.

Derartige Einsätze für Rotoren von Zentrifugen sind schon seit längerem bekannt, beispielsweise unter der Marke "*TACSI*" mit der Typenbezeichnung "*2G*" von der japanischen Firma Terumo, welche durch den Anmelder vertrieben werden. Unter der Bedienungsanleitung AB5400-30EN / Rev. 13 ist dieser Einsatz im Einzelnen zu entnehmen. Diese Einsätze dienen der automatisierten sowie der in die Zentrifuge integrierten Separation von z. B. roten Blutkörperchen und Blutplasma in einem Prozessbeutel eines Blutbeutelsystems. In einem solchen Einsatz wird ein zum Beispiel mit Spenderblut gefüllter Prozessbeutel eines Blutbeutelsystems verwendet. In dem Prozessbeutel befindet sich die beispielsweise vorgefilterte Blutspende. Das Blutbeutelsystem weist zum Beispiel eine oder mehrere Satellitenbeutel auf, welche leer oder mit einem Nährmedium vorgefüllt sein können. Die Satellitenbeutel und der Prozessbeutel sind untereinander mit Kunststoffschläuchen miteinander verbunden. Während der Zentrifugation setzen sich die roten Blutkörperchen - RBC - und das Blutplasma - PPP - entsprechend ihrer Dichte am äußeren Rand ab. Die Sedimentation erfolgt bei 2500 rpm - Umdrehungen pro Minute / revolutions per minute - des Rotors der Zentrifuge, so dass sich die dichteren roten Blutkörperchen außen im Prozessbeutel und weiter innen das weniger dichte Blutplasma anordnen.

Der Einsatz ist mit einem Gehäuse versehen, das einen Prozessbehälter für den Prozessbeutel als Teil des Gehäuses und weitere Behälter für die Satellitenbeutel umfasst. In dem Behälter für den Prozessbeutel ist ein Schieber angeordnet, der in Bezug auf eine Rotorachse radial gegen den Prozessbeutel ausfahrbar und wieder einfahrbar ist. Hierfür ist der Schieber mit einem Antrieb verbunden, der mit einer drehbaren Spindelmutter und einer Spindel zusammenwirkt. Die Spindelmutter ist dabei in dem Gehäuse des Einsatzes drehbar gelagert. Die Spindel durchgreift die Spindelmutter, ist in der Spindelmutter translatorisch bewegbar gelagert und mit dem Schieber drehfest verbunden.

Wie ausgeführt wurde, ist der Schieber in dem Prozessbehälter gelagert und wirkt auf den Prozessbeutel vorbestimmt ein, indem die Spindel und darüber der Schieber zwischen einer Ausgangsposition und einer Betriebsposition in Richtung auf die Rotorachse zu und von der Rotorachse wegbewegt wird. Die Spindelmutter ist in dem Gehäuse des Einsatzes über zumindest ein Lager drehbar gelagert und dient als Bewegungsumsetzer der über den Antrieb eingeleiteten Drehbewegung in eine translatorische Bewegung.

Da bei der Zentrifugation Wärme entsteht, dies sich aber nachteilig auf die roten Blutkörperchen und das Blutplasma auswirkt, da Blutkörper und Blutplasma ggfs. unbrauchbar werden, ist der Rotorraum der Zentrifuge, in der sich der Rotor mit dem Einsatz bzw. den Einsätzen befindet, gekühlt. Durch die Kühlung des Rotorraums soll daher auf alle Fälle ein Erwärmen der roten Blutkörperchen und des Blutplasmas verhindert werden.

Um das Blutplasma von den roten Blutkörperchen zu trennen, wird bei einer kleinen Drehzahl der Zentrifuge, beispielsweise im Bereich von 300-600 rpm, der Schieber über die Spindel gegen den Prozessbeutel gedrückt und dadurch das Blutplasma im Prozessbeutel aus diesem verdrängt. Über die angeschlossenen Kunststoffschläuche, welche insbesondere in und an dem Einsatz durch integrierte Schlauchklemmen gehalten werden, samt einer optischen Detektion, wird das Blutplasma in den oder die dazugehörigen Satellitenbeutel übergepresst. Die roten Blutkörperchen verbleiben dann beispielsweise in dem Prozessbeutel. Dieser Überpressvorgang mit dem Bewegen des Schiebers auf den Prozessbeutel geschieht unter Einwirkung der Zentrifugalkraft, also während des Betriebs der Zentrifuge, unter anderem zur Stabilisierung der einzelnen Schichten im Prozessbeutel.

Problematisch an den bekannten Einsätzen ist, dass die Prozessbeutel, aber auch die Satellitenbeutel, vor allem durch die bewegten Teile des Einsatzes kontaminiert werden können, beispielsweise durch im Betrieb entstehenden Abrieb, Rost und/oder durch Schmiermittel, so dass das Blutplasma sowie die roten Blutkörperchen nur noch eingeschränkt verwendet werden können oder unbrauchbar werden. Der Rost kann dabei durch die Kühlung und der damit verbundenen Kondensation der sich im Rotorraum befindlichen Luft hervorgerufen werden. Das Schmiermittel und der Abrieb können von den relativ zueinander bewegten Teilen kommen und in Folge der Zentrifugalkräfte auf das Beutelsystem aufgebracht werden. Infolge von Diffusionsvorgängen durch die Beutel können die Blutproben, die Blutkörperchen und das Blutplasma kontaminiert werden.

Der Erfindung liegt die Aufgabe zugrunde, einen Einsatz für einen Rotor einer Zentrifuge gemäß der im Oberbegriff des Patentanspruches 1 angegebenen Art derart weiterzubilden, dass eine Kontamination der Beutel des Beutelsystems, also der Prozessbeutel und ggfs. der Satellitenbeutel, nahezu ausgeschlossen werden kann.

Diese Aufgabe wird durch die kennzeichnenden Merkmale des Patentanspruches 1 in Verbindung mit seinen Oberbegriffsmerkmalen gelöst.

Die Unteransprüche bilden vorteilhafte Weiterbildungen der Erfindung.

Der Erfindung liegt die Erkenntnis zugrunde, dass das Lager für den Schieber eine wesentliche Quelle für eine mögliche Kontamination innerhalb des Einsatzes und des Beutelsystems ist und daher Maßnahmen zur Einschränkung der Kontamination hier anzusetzen haben. Es hat sich gezeigt, dass insbesondere durch ein schmierstofffreies Lager eine erhebliche Einschränkung der Kontamination erreicht werden kann.

Nach der Erfindung ist daher das Lager durch ein schmierstofffreies Lager mit einem äußeren an dem Gehäuse anliegenden Lagerring und einem an der Spindelmutter anliegenden Lagerring gebildet. Hierdurch wird auf einfache Weise verhindert, dass Schmierstoff austreten und die Beutel des Beutelsystems kontaminieren kann.

Um insbesondere auch die Kontamination durch Rost zu vermeiden, wird zudem ein rostfreies Lager verwendet. Die Kondensation hat dadurch keinen Einfluss mehr auf eine Rostbildung im Lager.

Da aber die Spindelmutter im Lager sich während der Zentrifugation bewegt, um das Blutplasma überzupressen, muss das Lager zusätzlich den bei der Zentrifugation auftretenden Kräften widerstehen. Es ist daher günstig, wenn das Lager durch ein Wälzlager gebildet ist, welches höhere Kräfte aufnehmen kann.

Wälzlager haben jedoch das Problem, dass Abrieb entsteht. Gemäß einer Ausführungsform der Erfindung ist daher das Lager durch ein Hybridlager gebildet. Hybridlager sind nahezu abriebsfrei. Die Wälzelemente können aus Keramik bestehen und der Außenring sowie der Innenring aus Edelstahl.

Um auch das Beutelsystem und den übrigen Einsatz vor geringfügigem Abrieb zu schützen, ist das Lager durch ein abgedichtetes, gekapseltes, insbesondere durch Kunststoff gekapseltes, Wälzlager gebildet.

Der Außenring und/oder der Innenring des Wälzlagers können einteilig oder mehrteilig ausgebildet sein. Für die in der Zentrifuge wirkenden Zentrifugalkräfte ist es im Hinblick auf die Stabilität des Lagers günstig, wenn der Außenring und/oder der Innenring des Wälzlagers einteilig ausgebildet sind.

Insbesondere bei der Verwendung lediglich eines Lagers ist es günstig, wenn das Lager als Vierpunktlager ausgebildet ist. Vierpunktlager sind beispielsweise einreihige Radial-Schrägkugellager, deren Laufbahnen so ausgebildet sind, dass Axialbelastungen in beide Richtungen aufgenommen werden können. Radiale Belastungen können nur bis zu einem Bruchteil der axialen Belastung aufgenommen werden.

Vorzugsweise weist das Lager neun Wälzelemente auf.

Gemäß einer Ausführungsform der Erfindung liegt die Drehachse des Lagers auf einer radialen Linie der Rotorachse. Auf diese Weise werden beim Prozess mögliche Unwuchten vermieden.

Um die Verbreitung von Rost, Abrieb und ähnlichem weiter einzuschränken, ist das Lager bereichsweise von dem Gehäuse, der Spindelmutter, einem Spannelement, einer Fixierplatte und einer weiteren Dichtung eingehaust.

Gemäß einer Ausführungsform der Erfindung schließt sich an das Lager auf der der Rotorachse abgewandten Seite des Lagers eine Außendichtung an, welche einen Lagerspalt zwischen Gehäuse und Spindelmutter abdichtet. Mit der Abdichtung wird ein Austreten von kontaminierten Stoffen durch die Außendichtung verhindert.

Vorzugsweise ist das Gehäuse mit einer Wand versehen, an welcher das Lager angeordnet ist. Zudem weist der Antrieb einen Motor und ein mit dem Motor verbundenes erstes Getriebe auf. Der Motor und das erste Getriebe sind auf der einen Seite der Wand angeordnet und der Schieber ist auf der anderen Seite der Wand angeordnet. Über die Wand kann auf einfache Weise der auf das Beutelsystem einwirkende Schieber von einem Großteil der bewegten Teile getrennt werden. Zudem dient die Wand als Aufnahme für den Außenring des Lagers und kann die Rückwand des Behälters bilden.

Damit die Antriebskraft vom Motor über das erste Getriebe auf die Spindelmutter übertragen werden kann, weist der Antrieb ein zweites Getriebe auf, welches sowohl mit dem ersten Getriebe als auch mit der Spindelmutter verbunden ist, wobei das zweite Getriebe auf der gleichen Seite wie der Schieber angeordnet ist.

Dabei kann der Antrieb gegenüber dem Schieber näher an der Rotorachse angeordnet sein. Dies ist auch insoweit günstig, da dann die bewegten Massen näher an der Rotorachse sind und sich dadurch eine stabilere Laufweise der Zentrifuge ergibt.

Für die Aufnahme des Lagers kann die Wand einen Absatz aufweisen. Dabei ist das Lager von der Seite, welche näher zur Rotorachse ist, in den Absatz mit dem Außenring des Lagers eingebracht. Hierdurch wird erreicht, dass konstruktiv bereits eine Trennung zwischen dem Lager auf der einen Seite und dem Schieber im Behälter für das Blutbeutelsystem auf der anderen Seite erfolgt.

Die Spindelmutter kann einen Absatz zur Aufnahme des Lagers aufweisen. Dabei wird das Lager von der Seite, welche näher zur Rotorachse ist, in den Absatz mit dem Innenring des Lagers eingebracht. Die Montage erfolgt somit von der dem Schieber abgewandten Seite der Wand. Die konstruktive Trennung der bewegten Teile des Antriebs mit dem Lager auf der einen Seite und des Schiebers auf der anderen Seite wird hierdurch auf einfache Weise ermöglicht.

Vorzugsweise ist der Außenring auf der dem Absatz der Wand entfernt gelegenen Seite des Gehäuses durch ein mit der Wand des Gehäuses, insbesondere lösbar, verbundenes Fixierelement, insbesondere eine Fixierplatte, in axialer Richtung mit der Wand des Gehäuses verbunden.

Der Innenring kann auf der dem Absatz der Spindelmutter entfernt gelegenen Seite durch ein Spannelement, insbesondere ein auf die Spindelmutter aufgebrachter Spannring oder eine auf der Spindelmutter aufgebrachte Gewindemutter, in axialer Richtung mit der Spindelmutter verbunden sein.

Zudem kann zwischen Fixierelement und Spannelement eine Innendichtung vorgesehen sein, welche den Lagerspalt zwischen Fixierplatte und Spannmutter abdichtet. Hierdurch wird eine weitere Separation von kontaminierenden Stoffen gegenüber dem Behälter mit dem Schieber auf einfache Weise ermöglicht.

Weitere Vorteile, Merkmale und Anwendungsmöglichkeiten der vorliegenden Erfindung ergeben sich aus der nachfolgenden Beschreibung in Verbindung mit den in den Zeichnungen dargestellten Ausführungsbeispielen.

In der Beschreibung, in den Ansprüchen und in der Zeichnung werden die in der unten aufgeführten Liste der Bezugszeichen verwendeten Begriffe und zugeordneten Bezugszeichen verwendet. In der Zeichnung bedeutet:
- Fig. 1: eine perspektivische Ansicht von schräg oben auf eine Zentrifuge mit geöffnetem Deckel und in den Rotor eingebrachten Einsätze nach der Erfindung;
- Fig. 2: eine perspektivische Detailansicht von schräg oben auf die in den Rotor eingebrachten Einsätze von Fig. 1;
- Fig. 3: eine perspektivische Ansicht auf einen aus dem Rotor entnommenen Einsatz;
- Fig. 4: eine perspektivische Ansicht auf den Einsatz von Fig. 3 mit einem Teilschnitt durch den inneren Bereich des Einsatzes im Bereich der Innenabdeckung;
- Fig. 5: eine perspektivische Ansicht auf den Einsatz von Fig. 3 ohne eine Innenabdeckung und weiterer Einbauten;
- Fig. 6: eine Seitenschnittansicht durch den Einsatz entlang einer Drehachse einer Spindelmutter des Einsatzes mit einem eingefahrenen Schieber und einem in einen Behälter eingebrachten Prozessbeutel, und
- Fig. 7: eine Seitenschnittansicht wie in Fig. 6, jedoch mit ausgefahrenem Schieber.

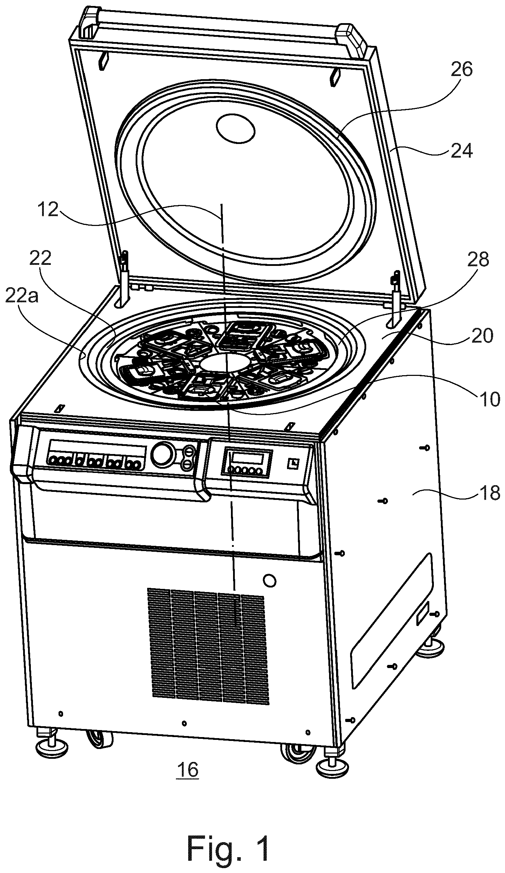

In den Figuren 1 bis 7 ist eine Ausführungsform eines Einsatzes 10 für einen um eine Rotorachse 12 drehbaren Rotor 14 einer Zentrifuge 16 dargestellt.

Wie der Fig. 1 zu entnehmen ist, ist die Zentrifuge 16 mit einem geschlossenen Gehäuse 18 versehen, welches an seiner oberen Deckenwand 20 eine kreisrunde Beladungsöffnung 22 aufweist. Die Beladungsöffnung 22 kann über einen an das Gehäuse 18 angelenkten Deckel 24 verschlossen werden. Der Deckel 24 weist hierfür einen an die Beladungsöffnung 22 angepassten und zu dieser Beladungsöffnung 22 ausgerichtet angeordneten Dichtungsring 22a auf. Der Dichtungsring 22a greift bei geschlossenem Deckel 24 in einen Ringabsatz 26 der Beladungsöffnung 22 ein und schließt darüber die Beladungsöffnung 22 dichtend ab.

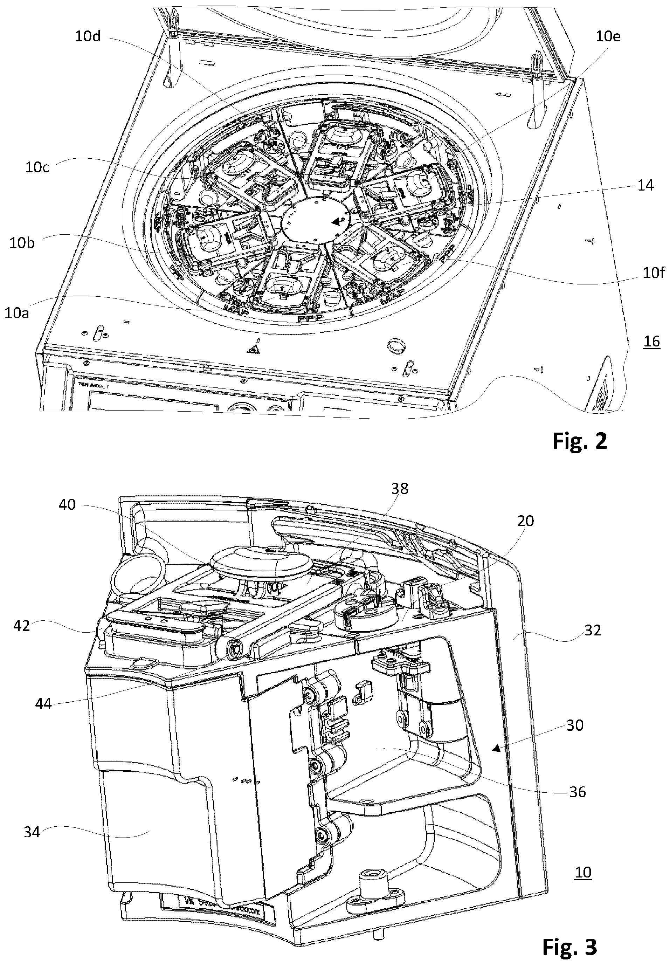

Die Beladungsöffnung 22 ermöglicht bei geöffnetem Deckel 24 eine Beladung des Rotors 14, in dem sechs erfindungsgemäße Einsätze 10a bis 10f eingebracht sind, siehe Fig. 2. Der Rotor 14 mit den Einsätzen 10 ist in einem gekühlten Rotorraum 28 angeordnet. Alle sechs Einsätze 10a bis 10f sind gleich aufgebaut, so dass die Beschreibung eines Einsatzes 10 für alle Einsätze 10a bis 10f gilt.

Wie den Fig. 3 bis 7 zu entnehmen ist, umfasst ein Einsatz 10 ein Gehäuse 30. Das Gehäuse 30 ist mit einer Außenwand 32 und einer Innenabdeckung 34 versehen, welche das Gehäuse radial nach außen bzw. nach innen begrenzen. Zudem umfasst das Gehäuse 30 einen Prozessbehälter 36, der über einen Prozessbehälterdeckel 38 mit einer Handhabe 40 verschlossen ist. Der Prozessbehälterdeckel 38 ist im oberen Bereich des Prozessbehälters 36 über ein erstes und ein zweites Gelenk 42, 44 mit einer oberen Einsatzwand 46 des Gehäuses 30 des Einsatzes 10 verbunden.

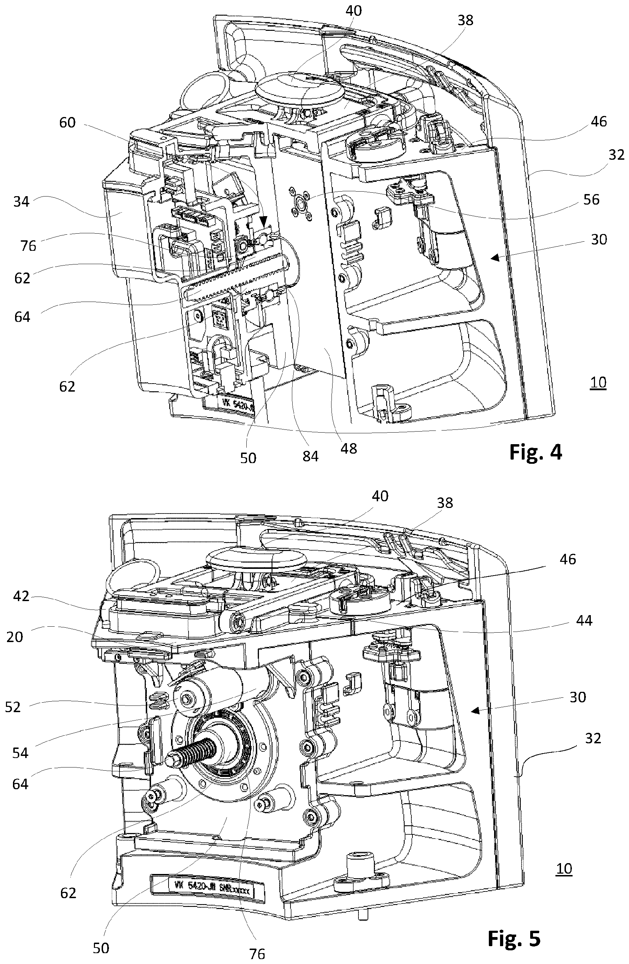

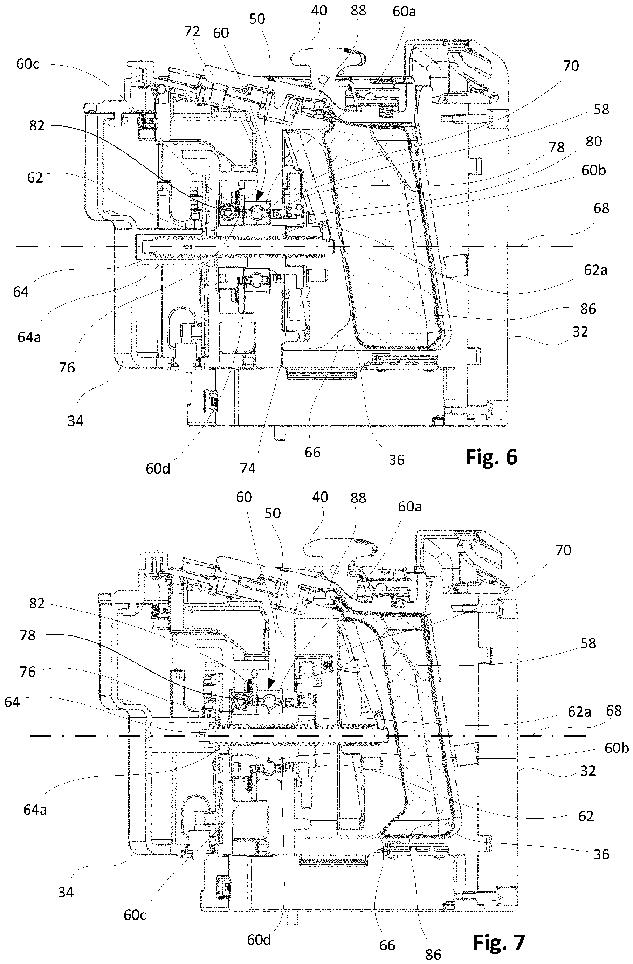

An eine Rückwand 48 des Prozessbehälters 36 schließt sich eine Tragwand 50 an, die im Bereich der Rückwand 48 des Prozessbehälters 36 seitlich mit dem Gehäuse 30 verschraubt ist, siehe Fig. 4 und Fig. 5. Die Rückwand 48 und die Tragwand 50 können auch identisch sein, wie dies in den Fig. 6 und 7 dargestellt ist.

An der Tragwand 50 sind ein Motor 52 und ein erstes Getriebe 54 fest angeordnet. Über eine Öffnung 56, siehe Fig. 4, erstreckt sich eine hier nicht dargestellte Antriebsspindel und verbindet das erste Getriebe 54 mit einem zweiten Getriebe 58, siehe Fig. 6 und 7. Das zweite Getriebe 58 ist mit einer in der Tragwand 50 über ein Lager 60 drehbar gelagerten Spindelmutter 62 verbunden, so dass über den Motor 52, das erste Getriebe 54 der hier nicht dargestellten Antriebsspindel und das zweite Getriebe 58 die Spindelmutter 62 angetrieben werden kann.

In der Spindelmutter 62 ist eine axial verschiebliche Spindel 64 gelagert, welche an ihrer Außenseite ein Gewinde 64a aufweist, in welche ein Innengewinde 62a der Spindelmutter 62 eingreift. Die Spindel 64 ist drehfest mit einem Schieber 66 verbunden. Mit Drehen der Spindelmutter 62 werden die Spindel über das ineinandergreifende Innengewinde 62a der Spindelmutter 62 sowie das Außengewinde 64a der Spindel 64 und somit auch der Schieber 66 axial verschoben. Je nach Drehrichtung erfolgt eine axiale Verschiebung der Spindel 64 von der Rotorachse 12 weg oder auf die Rotorachse 12 zu. Die Drehachse der Spindelmutter 62 und die Längsachse der Spindel 64 bilden eine gemeinsame Achse 68, welche sich radial von der Rotorachse 12 weg erstreckt.

Die Spindelmutter 62 dient im Zusammenwirken mit der Spindel 64 somit als Bewegungsumsetzer von einer durch den Motor 52, dem ersten Getriebe 54 und dem zweiten Getriebe 58 in die Spindelmutter 62 eingeleiteten Rotationsbewegung in eine Translationsbewegung der Spindel 64 und somit des Schiebers 66.

Die Spindel 64 ragt weit in den durch die Innenabdeckung 34 begrenzten Raum hinein und somit aus der Spindelmutter 62 heraus, da der Schieber 66 sich in seiner Ausgangsposition befindet, siehe Fig. 4, Fig. 5 und Fig. 6.

Bei dem Lager 60 handelt es sich um ein schmierstofffreies, rostfreies Wälzlager, nämlich ein Vierpunkt-Hybridlager. Das Lager 60 liegt an einem Absatz 70 der Tragwand 50 mit seinem Außenring 60a an. Der Außenring 60a ist in dem Absatz 70 durch eine mit der Tragwand 50 an der von dem Prozessbehälter 36 abgewandten Seite befestigte Fixierplatte 72 in axialer Richtung spielfrei festgelegt. Weiterhin liegt das Lager 60 mit seinem Innenring 60b an einem Außenabsatz 74 der Spindelmutter 62 an. Über einen Spannring 76 ist der Innenring 60b fest in Richtung Außenabsatz 74 gespannt.

In den Fig. 6 und Fig. 7 wurde für das Lager 60 ein geteilter Innenring 60b verwendet. Günstiger ist es jedoch, einen einteiligen Innenring 60b zu verwenden.

Zwischen dem Außenring 60a und dem Innenring 60b sind neun Wälzelemente in Form von Wälzkugeln 60c eingebracht. Das Lager 60 ist mit einem links und rechts der Wälzkugeln 60c angeordneten sowie zwischen dem Außenring 60a und dem Innenring 60b angeordneten Lagergehäuse 60d abgeschlossen. Das Lagergehäuse 60d besteht aus Kunststoff und dichtet das Lager 60 ab, so dass kein Abrieb nach außen treten kann.

Der Außenring 60a und der Innenring 60b sind aus rostfreiem Edelstahl gebildet, insbesondere X1005CrMo17. Die Wälzkugeln 60 c sind aus Keramik gebildet, insbesondere aus Si3N4.

Wie insbesondere der Fig. 6 und der Fig. 7 zu entnehmen ist, wird ein im Bereich des Absatzes 70 der Tragwand 50 sich befindlicher Lagerspalt 78 durch eine Außendichtung 80 abgedichtet. Auf diese Weise ist das gekapselte Lager 60 durch die Tragwand 50, die Außendichtung 80, die Spindelmutter 62, sowie den Spannring 76 und die Fixierplatte 72 noch einmal eingehaust/gekapselt.

Zwischen der Fixierplatte 72, dem Spannring 76 und dem Innenring 60b kann zudem eine weitere Dichtung 82 vorgesehen sein.

Der Absatz 70 der Tragwand 50 sowie der Außenabsatz 74 der Spindelmutter 62 sind so angeordnet, dass die Montage des Lagers 60 von der der Rotorachse 12 nächstliegenden Seite der Tragwand 50 erfolgt. Die Spindelmutter 62 hingegen wird von der Seite der Tragwand 50 und der Rückwand 48 des Prozessbehälters 36 in eine zugeordnete Öffnung 84 eingeführt, welche von der Rotorachse 12 entfernt liegt.

Die Innenabdeckung 34 wird von der zur Rotorachse 12 weisenden Seite der Tragwand 50 auf diese gesteckt und über einen hier nicht näher dargestellten Klickmechanismus mit dieser lösbar verbunden.

In den Prozessbehälter 36 ist in Fig. 6 und 7 jeweils ein Prozessbeutel 86 eines Beutelsystems eingebracht. Dieser Prozessbeutel 86 ist mit Spenderblut gefüllt. Fig. 6 zeigt den Schieber 66 in seiner Ausgangsposition. Der Prozessbeutel 86 ist über Kunststoffschläuche 88 mit hier nicht dargestellten Satellitenbeuteln verbunden. In dieser Ausgangsposition des Schiebers 66 startet die Zentrifugation durch Drehen des Rotors 14. Dabei setzen sich die roten Blutkörperchen - RBC - und das Blutplasma - PPP - entsprechend ihrer Dichte am äußeren Rand ab. Die Sedimentation erfolgt bei 2500 rpm - Umdrehungen pro Minute / revolutions per minute - des Rotors 14 der Zentrifuge 16, so dass sich die dichteren roten Blutkörperchen außen im Prozessbeutel 86 und weiter innen das nicht so dichte Blutplasma anordnen. Ist die vollständige Trennung erfolgt, wird das Blutplasma aus dem Prozessbeutel 86 entfernt.

Hierfür wird der Motor 52 gestartet und treibt über das erste Getriebe 54 und das zweite Getriebe 58 die Spindelmutter 62 an. Daraufhin wird die Spindel 64, welche mit dem Schieber 66 verbunden ist, translatorisch aus der in Fig. 6 dargestellten Ausgangsposition in Richtung der in Fig. 7 dargestellten Betriebsposition bewegt und mit der Spindel 64 auch der Schieber 66.

Um das Blutplasma aus dem Prozessbeutel 86 mit den roten Blutkörperchen, jedoch ohne diese, herauszubewegen, wird bei einer kleinen Drehzahl der Zentrifuge 16, beispielsweise im Bereich von 300-600 rpm, der Schieber 66 über die Spindel 64 wie beschrieben sukzessive gegen den Prozessbeutel 86 gedrückt. Dadurch wird das Blutplasma im Prozessbeutel 86 aus diesem verdrängt. Über den angeschlossenen Kunststoffschlauch 88, welcher in und an dem Einsatz durch integrierte, hier nicht dargestellte Schlauchklemmen gehalten wird, samt einer optischen Detektion, welche mit einem Regler/Steuergerät zusammenwirkt, wird das Blutplasma in den oder die dazugehörigen Satellitenbeutel übergepresst. Die roten Blutkörperchen verbleiben dann in dem Prozessbeutel. Dieser Überpressvorgang mit dem Bewegen des Schiebers 66 in den Satellitenbeutel geschieht unter Einwirkung der Zentrifugalkraft, also während des Betriebs der Zentrifuge 16, unter anderem zur Stabilisierung der einzelnen Schichten im Prozessbeutel 86.

Nach der Erfindung ist das Lager 60, als potenzielle Quelle von Kontaminationsstoffen, weitestgehend so gestaltet, dass diese nicht vorhanden sind und nicht entstehen können. Zudem ist das Lager 60 stufenweise eingehaust/gekapselt, so dass auf alle Fälle vermieden wird, dass Kontaminationsstoffe in den Prozessbehälter 36 und somit auf den Prozessbeutel 86 gelangen können. Eine Kontamination der in dem Prozessbeutel 86 befindlichen Stoffe, beispielsweise Blutspenden, wird dadurch auf einfache Weise effektiv verhindert.

### Bezugszeichenliste

- 10: Einsatz
- 10a: erster Einsatz
- 10b: zweiter Einsatz
- 10c: dritter Einsatz
- 10d: vierter Einsatz
- 10e: fünfter Einsatz
- 10f: sechster Einsatz
- 12: Rotorachse
- 14: Rotor
- 16: Zentrifuge
- 18: Gehäuse
- 20: obere Deckenwand des Gehäuses der Zentrifuge
- 22: Beladungsöffnung
- 22a: Dichtungsring
- 24: Deckel
- 26: Ringabsatz
- 28: Rotorraum
- 30: Gehäuse des Einsatzes 10
- 32: Außenwand
- 34: Innenabdeckung
- 36: Prozessbehälter
- 38: Prozessbehälterdeckel
- 40: Handhabe
- 42: erstes Gelenk
- 44: zweites Gelenk
- 46: obere Einsatzwand 46 des Gehäuses 30 des Einsatzes 10
- 48: Rückwand des Prozessbehälters 36
- 50: Tragwand
- 52: Motor
- 54: erstes Getriebe
- 56: Öffnung
- 58: zweites Getriebe
- 60: Lager
- 60a: Außenring des Lagers 60
- 60b: Innenring des Lagers 60
- 60c: Wälzelemente des Lagers 60
- 60d: Lagergehäuse des Lagers 60
- 62: Spindelmutter
- 62a: Innengewinde der Spindelmutter 62
- 64: Spindel
- 64a: Außengewinde der Spindel 64
- 66: Schieber
- 68: Achse
- 70: Absatz der Tragwand 50
- 72: Fixierplatte
- 74: Außenabsatz der Spindelmutter 62
- 76: Spannring
- 78: Lagerspalt
- 80: Außendichtung
- 82: weitere Dichtung, Innendichtung
- 84: Öffnung für Spindelmutter in der Tragwand 50 und der Rückwand 48 des Prozessbehälter 36
- 86: Prozessbeutel
- 88: Kunststoffschlauch

## Patentansprüche

1. Einsatz (10) für einen um eine Rotorachse (12) drehbaren Rotor (14) einer Zentrifuge (16) mit einem Gehäuse (30), das zumindest einen Behälter (36) für Beutel (86) aufweist, insbesondere Blutbeutel, mit einem Schieber (66), der mit einem Antrieb (52, 54, 58), einer drehbaren Spindelmutter (62) und einer Spindel (64) zusammenwirkt, wobei die Spindelmutter (62) in dem Gehäuse (30) drehbar gelagert ist, mit dem Antrieb (52, 54, 58) verbunden ist, und die Spindel (64) in der Spindelmutter (62) translatorisch verschiebbar gelagert ist und mit dem Schieber (66) verbunden ist, wobei der Schieber (66) in dem Behälter (36) angeordnet ist und auf den Beutel (86) vorbestimmt einwirkt, indem die Spindel (64) zwischen einer Ausgangsposition und einer Betriebsposition bewegbar ist, wobei die Spindelmutter (62) in dem Gehäuse (30) über zumindest ein Lager (60) drehbar gelagert ist, **dadurch gekennzeichnet, dass** das Lager (60) durch ein schmierstofffreies Lager (60) mit einem äußeren an dem Gehäuse (30) anliegenden Lagerring (60a) und einem an der Spindelmutter (62) anliegenden Lagerring (60b) gebildet ist.

2. Einsatz (10) nach Anspruch 1, **dadurch gekennzeichnet, dass** das Lager (60) ein rostfreies Lager ist.

3. Einsatz (10) nach einem der vorangehenden Ansprüche, **dadurch gekennzeichnet, dass** das Lager (60) ein Wälzlager ist.

4. Einsatz (10) nach Anspruch 3, **dadurch gekennzeichnet, dass** das Lager (60) durch ein Hybridlager gebildet ist.

5. Einsatz (10) nach einem der Ansprüche 3 oder 4, **dadurch gekennzeichnet, dass** das Lager (60) durch ein abgedichtetes, gekapseltes, insbesondere durch Kunststoff gekapseltes, Wälzlager gebildet ist.

6. Einsatz (10) nach einem der Ansprüche 3 bis 5, **dadurch gekennzeichnet, dass** der Außenring (60a) und/oder der Innenring (60b) des Wälzlagers (60) einteilig gebildet sind.

7. Einsatz (10) nach einem der Ansprüche 3 bis 6, **dadurch gekennzeichnet, dass** das Lager (60) ein Vierpunktlager ist.

8. Einsatz (10) nach einem der Ansprüche 3 bis 7, **dadurch gekennzeichnet, dass** das Lager (60) neun Wälzelemente (60c) aufweist.

9. Einsatz (10) nach einem der vorangehenden Ansprüche, **dadurch gekennzeichnet, dass** die Drehachse (68) des Lagers (60) auf einer radialen Linie der Rotorachse (12) liegt.

10. Einsatz (10) nach einem der vorangehenden Ansprüche, **dadurch gekennzeichnet, dass** das Lager (60) bereichsweise von dem Gehäuse (30), der Spindelmutter (62), einem Spannelement wie einem Spannring (76), einem Fixierelement wie einer Fixierplatte (72) und einer weiteren Dichtung (82) eingehaust ist.

11. Einsatz (10) nach einem der vorangehenden Ansprüche, **dadurch gekennzeichnet, dass** sich an das Lager (60) auf der der Rotorachse (12) abgewandten Seite des Lagers (60) eine Außendichtung (80) anschließt, welche einen Lagerspalt (78) zwischen Gehäuse (30) und Spindelmutter (62) abdichtet.

12. Einsatz (10) nach einem der vorangehenden Ansprüche, **dadurch gekennzeichnet, dass** das Gehäuse (30) eine Wand (48, 50) aufweist, in welcher das Lager (60) angeordnet ist, der Antrieb einen Motor (52) und ein mit dem Motor (52) verbundenes erstes Getriebe (54) aufweist, der Motor (52) und das erste Getriebe (54) auf der einen Seite der Wand (48, 50) angeordnet sind und der Schieber (66) auf der anderen Seite der Wand (48, 50) angeordnet ist.

13. Einsatz (10) nach Anspruch 12, **dadurch gekennzeichnet, dass** der Antrieb ein zweites Getriebe (58) aufweist, welches sowohl mit dem ersten Getriebe (54) als auch mit der Spindelmutter (62) verbunden ist, wobei das zweite Getriebe (58) auf der gleichen Seite relativ zur Wand (48, 50) wie der Schieber (66) angeordnet ist.

14. Einsatz (10) nach Anspruch 12 oder 13, **dadurch gekennzeichnet, dass** der Antrieb (52, 54, 58) gegenüber dem Schieber (66) näher an der Rotorachse (12) angeordnet ist.

15. Einsatz (10) nach einem der Ansprüche 12 bis 14, **dadurch gekennzeichnet, dass** die Wand (50) einen Absatz (70) zur Aufnahme des Lagers (60) aufweist, und dass das Lager (60) von der Seite, welche näher zur Rotorachse (12) ist, in den Absatz (70) mit dem Außenring (60a) des Lagers (60) eingebracht ist.

16. Einsatz (10) nach Anspruch 15, **dadurch gekennzeichnet, dass** die Spindelmutter (62) einen Absatz (74) zur Aufnahme des Lagers (60) aufweist, und dass das Lager (60) von der Seite, welche näher zur Rotorachse (12) ist, in den Absatz (74) mit dem Innenring (60b) des Lagers (60) eingebracht ist.

17. Einsatz (10) nach Anspruch 15 oder 16, **dadurch gekennzeichnet, dass** der Außenring (60a) auf der dem Absatz (70) der Wand (48, 50) entfernt gelegenen Seite des Gehäuses (18) durch ein mit der Wand (50) des Gehäuses (18), insbesondere lösbar, verbundenes Fixierelement, insbesondere eine Fixierplatte (72), in axialer Richtung mit der Wand (50) des Gehäuses (30) verbunden ist.

18. Einsatz (10) nach Anspruch 16 oder 17, **dadurch gekennzeichnet, dass** der Innenring (60b) auf der dem Absatz (74) der Spindelmutter (62) entfernt gelegenen Seite durch ein Spannelement (76), insbesondere einen auf die Spindelmutter (62) aufgebrachten Spannring (76), in axialer Richtung mit der Spindelmutter (62) verbunden ist.

19. Einsatz (10) nach Anspruch 17 und 18, **dadurch gekennzeichnet, dass** zwischen Fixierelement (72) und Spannelement eine Innendichtung vorgesehen ist, welche den Lagerspalt (78) zwischen Fixierplatte (72) und Spannelement (76) abdichtet.

## Claims

1. Insert (10) for a rotor (14) of a centrifuge (16), which rotor is rotatable about a rotor axis (12), comprising: a housing (30) that has at least one container (36) for bags (86), in particular blood bags, a slider (66) that cooperates with a drive (52, 54, 58), a rotatable spindle nut (62) and a spindle (64), which spindle nut (62) is rotatably mounted in the housing (30), is connected to the drive (52, 54, 58), and which spindle (64) is translationally movably mounted in the spindle nut (62) and is connected to the slider (66), which slider (66) is located inside the container (36) and acts on the bag (86) in a predetermined manner, in that the spindle (64) can be moved between a starting position and an operating position thereof, with the spindle nut (62) being rotatably mounted in the housing (30) via at least one bearing (60), **characterized in that** the bearing (60) is constituted by a lubricant-free bearing (60) having an outer bearing ring (60a) resting on the housing (30) and an inner bearing ring (60b) resting on the spindle nut (62).

2. Insert (10) according to claim 1, **characterized in that** the bearing (60) is a stainless bearing.

3. Insert according to any one of the preceding claims, **characterized in that** the bearing (60) is a roller bearing.

4. Insert (10) according to claim 3, **characterized in that** the bearing (60) is constituted by a hybrid bearing.

5. Insert (10) according to any one of claims 3 or 4 above, **characterized in that** the bearing (60) is constituted by a sealed, encapsulated roller bearing, which is in particular encapsulated by a plastic material.

6. Insert (10) according to any one of claims 3 to 5 above, **characterized in that** the outer ring (60a) and/or the inner ring (60b) of the roller bearing (60) are formed as one piece.

7. Insert (10) according to any one of claims 3 to 6 above, **characterized in that** the bearing (60) is a four-point contact bearing.

8. Insert (10) according to any one of claims 3 to 7 above, **characterized in that** the bearing (60) has nine roller elements (60c).

9. Insert (10) according to any one of the preceding claims, **characterized in that** the rotational axis (68) of the bearing (60) is on a radial line of the rotor axis (12).

10. Insert (10) according to any one of the preceding claims, **characterized in that** some areas of the bearing (60) are enclosed by the housing (30), the spindle nut (62), a clamping element such as a clamping ring (76), a fixing element such as a fixing plate (72) and an additional seal (82).

11. Insert (10) according to any one of the preceding claims, **characterized in that** an outer seal (80) adjoins the bearing (60) on the side of the bearing (60) that faces away from the rotor axis (12), which outer seal (80) seals a bearing gap (78) between the housing (30) and the spindle nut (62).

12. Insert (10) according to any one of the preceding claims, **characterized in that** the housing (30) comprises a wall (48, 50) in which the bearing (60) is arranged, the drive comprises a motor (52) and a first gear (54) connected to the motor (52), which motor (52) and first gear (54) are located on one side of the wall (48, 50) and which slider (66) is located on the other side of the wall (48, 50).

13. Insert (10) of claim 12, **characterized in that** the drive comprises a second gear (58) connected to both the first gear (54) and the spindle nut (62), which second gear (58) is located on the same side relative to the wall (48, 50) as the slider (66).

14. Insert (10) according to any one of claims 12 or 13 above, **characterized in that** the drive (52, 54, 58) is located closer to the rotor axis (12) compared to the slider (66).

15. Insert (10) according to any one of claims 12 to 14 above, **characterized in that** a recess (70) for receiving the bearing (60) is made in the wall (50), and that the bearing (60) is inserted into the recess (70) with the outer ring (60a) of the bearing (60) from the side that is closer to the rotor axis (12).

16. Insert (10) according to claim 15, **characterized in that** a recess (74) for receiving the bearing (60) is made in the spindle nut (62), and that the bearing (60) is inserted into the recess (74) with the inner ring (60b) of the bearing (60) from the side which is closer to the rotor axis (12).

17. Insert (10) according to any one of claims 15 or 16 above, **characterized in that** on the side of the housing (18) remote from the recess (70) in the wall (48, 50), the outer ring (60a) is connected to the wall (50) of the housing (30) in the axial direction by a fixing element, in particular a fixing plate (72) that is connected to the wall (50) of the housing (18), in particular in a detachable manner.

18. Insert (10) according to any one of claims 16 or 17 above, **characterized in that** on the side remote from the recess (74) in the spindle nut (62), the inner ring (60b) is connected to the spindle nut (62) in the axial direction by a clamping element (76), in particular a clamping ring (76) that is mounted on the spindle nut (62).

19. Insert (10) according to any one of claims 17 and 18 above, **characterized in that** an inner seal is fitted between the fixing element (72) and the clamping element, said seal acting to seal the bearing gap (78) between the fixing plate (72) and the clamping element (76).

## Revendications

1. Insert (10) pour un rotor (14) d'une centrifugeuse (16) rotatif autour d'un axe de rotor (12) avec un logement (30) qui présente au moins un contenant (36) pour poche (86), en particulier une poche de sang, avec un coulisseau (66) qui coopère avec un entraînement (52, 54, 58), un écrou de broche (62) rotatif et une broche (64), dans lequel l'écrou de broche (62) est monté en rotation dans le logement (30), est relié à l'entraînement (52, 54, 58), et la broche (64) est montée de façon à pouvoir coulisser par translation dans l'écrou de broche (62) et est reliée au coulisseau (66), dans lequel le coulisseau (66) est agencé dans le contenant (36) et agit de façon prédéterminée sur la poche (86), en ce que la broche (64) peut être déplacée entre une position de sortie et une position de fonctionnement, dans lequel l'écrou de broche (62) est monté en rotation dans le logement (30) via au moins un roulement (60), **caractérisé en ce que** le roulement (60) est formé par un roulement (60) exempt de lubrifiant avec une bague de roulement (60a) extérieure reposant contre le logement (30) et une bague de roulement (60b) reposant contre l'écrou de broche (62).

2. Insert (10) selon la revendication 1, **caractérisé en ce que** le roulement (60) est un roulement inoxydable.

3. Insert (10) selon l'une des revendications précédentes, **caractérisé en ce que** le roulement (60) est un palier à rouleau.

4. Insert (10) selon la revendication 3, **caractérisé en ce que** le roulement (60) est formé par un roulement hybride.

5. Insert (10) selon l'une des revendications 3 ou 4, **caractérisé en ce que** le roulement (60) est formé par un palier à rouleau étanche, encapsulé, en particulier encapsulé par une matière plastique.

6. Insert (10) selon l'une des revendications 3 à 5, **caractérisé en ce que** la bague extérieure (60a) et/ou la bague intérieure (60b) du palier à rouleau (60) est formée en une seule pièce.

7. Insert (10) selon l'une des revendications 3 à 6, **caractérisé en ce que** le roulement (60) est un roulement à quatre points.

8. Insert (10) selon l'une des revendications 3 à 7, **caractérisé en ce que** le roulement (60) présente neuf éléments de rouleau (60c).

9. Insert (10) selon l'une des revendications précédentes, **caractérisé en ce que** l'axe de rotation (68) du roulement (60) se trouve sur une ligne radiale de l'axe de rotor (12).

10. Insert (10) selon l'une des revendications précédentes, **caractérisé en ce que** le roulement (60) est par endroits logé par le logement (30), l'écrou de broche (62), un élément de serrage telle une bague de serrage (76), un élément de fixation telle une plaque de fixation (72) et un autre joint (82).

11. Insert (10) selon l'une des revendications précédentes, **caractérisé en ce qu'**un joint extérieur (80) se raccorde au roulement (60) sur le côté du roulement (60) opposé à l'axe de rotor (12), lequel joint extérieur étanchéifie un interstice de roulement (78) entre le logement (30) et l'écrou de broche (62).

12. Insert (10) selon l'une des revendications précédentes, **caractérisé en ce que** le logement (30) présente une paroi (48, 50) dans laquelle est agencé le roulement (60), l'entraînement présente un moteur (52) et une première transmission (54) reliée au moteur (52), le moteur (52) et la première transmission (54) sont agencés sur le un côté de la paroi (48, 50) et le coulisseau (66) est agencé sur l'autre côté de la paroi (48, 50).

13. Insert (10) selon la revendication 12, **caractérisé en ce que** l'entraînement présente une seconde transmission (58), laquelle est reliée tant à la première transmission (54) qu'à l'écrou de broche (62), dans lequel la seconde transmission (58) est agencée sur le même côté que le coulisseau (66) par rapport à la paroi (48, 50).

14. Insert (10) selon la revendication 12 ou 13, **caractérisé en ce que** l'entraînement (52, 54, 58) est agencé plus proche de l'axe de rotor (12) par rapport au coulisseau (66).

15. Insert (10) selon l'une des revendications 12 à 14, **caractérisé en ce que** la paroi (50) présente un épaulement (70) pour la réception du roulement (60), et **en ce que** le roulement (60) est introduit dans l'épaulement (70) avec la bague extérieure (60a) du roulement (60) depuis le côté qui est le plus proche de l'axe de rotor (12).

16. Insert (10) selon la revendication 15, **caractérisé en ce que** l'écrou de broche (62) présente un épaulement (74) pour la réception du roulement (60), et **en ce que** le roulement (60) est introduit dans l'épaulement (74) avec la bague intérieure (60b) du roulement (60) depuis le côté qui est le plus proche de l'axe de rotor (12).

17. Insert (10) selon la revendication 15 ou 16, **caractérisé en ce que** la bague extérieure (60a) sur le côté du logement (18) situé à distance de l'épaulement (70) de la paroi (48, 50) est reliée, dans la direction axiale, à la paroi (50) du logement (30) par un élément de fixation, en particulier une plaque de fixation (72), relié, en particulier de façon amovible, à la paroi (50) du logement (18).

18. Insert (10) selon la revendication 16 ou 17, **caractérisé en ce que** la bague intérieure (60b) sur le côté situé à distance de l'épaulement (74) de l'écrou de broche (62) est reliée, dans la direction axiale, à l'écrou de broche (62) par un élément de serrage (76), en particulier une bague de serrage (76) apportée sur l'écrou de broche (62).

19. Insert (10) selon la revendication 17 et 18, **caractérisé en ce qu'**un joint intérieur est prévu entre l'élément de fixation (72) et l'élément de serrage, lequel joint intérieur étanchéifie l'interstice de roulement (78) entre la plaque de fixation (72) et l'élément de serrage (76).
